# EUROPEAN PATENT SPECIFICATION

(11) **EP 1 685 794 B1**
(45) Date of publication and mention of the grant of the patent: **27.02.2013**
(21) Application number: 04818864.3
(22) Date of filing: 11.11.2004
(51) Int. Cl.: A61B 5/00, A61B 5/05

(54) **INPUT DEVICE AND INPUT METHOD**
EINGABEVORRICHTUNG UND EINGABEVERFAHREN
DISPOSITIF D'ENTREE ET PROCEDE D'ENTREE

(30) Priority: 18.11.2003 JP 2003388241; 10.12.2003 JP 2003412273
(43) Date of publication of application: 02.08.2006
(73) Proprietor: Sony Corporation, Tokyo 141-0001 (JP)
(72) Inventor: MIYAJIMA, Yasushi c/o Sony Corporation, Shinagawa-ku, Tokyo 141-0001 (JP); SAKO, Yoichiro c/o Sony Corporation, Shinagawa-ku, Tokyo 141-0001 (JP); TERAUCHI, Toshiro c/o Sony Corporation, Shinagawa-ku, Tokyo 141-0001 (JP); INOUE, Makoto c/o Sony Corporation, Shinagawa-ku, Tokyo 141-0001 (JP); SHIRAI, Katsuya c/o Sony Corporation, Shinagawa-ku, Tokyo 141-0001 (JP); ASUKAI, Masamichi c/o Sony Corporation, Shinagawa-ku, Tokyo 141-0001 (JP); TAKAI, Motoyuki c/o Sony Corporation, Shinagawa-ku, Tokyo 141-0001 (JP); MAKINO, Kenichi c/o Sony Corporation, Shinagawa-ku, Tokyo 141-0001 (JP)
(74) Representative: Nicholls, Michael John
(86) International application number: PCT/JP2004/016769
(87) International publication number: WO 2005/048832

(56) References cited:
- WO-A1-01/76220
- JP-A- 2 166 493
- JP-A- 2 166 493
- JP-A- 7 299 040
- JP-A- 2000 358 088
- JP-A- 2000 358 088
- JP-A- 2002 233 560
- JP-A- 2002 233 560
- JP-A- 2003 144 392
- JP-A- 2003 144 392
- JP-A- 2003 225 214
- JP-A- 2003 225 214

## Description

The present invention relates to an input device, an input method and an electronic equipment which are adapted for detecting bio-information (bioinformation) of user, and relates to an input device, an input method and an electronic equipment which are adapted to have ability to detect bio-information in the ordinary process of equipment use by user. Further, the present invention relates to an input device, an input method and an electronic equipment which are adapted for eliminating unevenness of detection values based on way of use of user to have ability to perform stable detection.

In the so-called Internet society in which communication network is used to perform transmission of information, technologies effective for more strengthening particularly information security and/or network security are required. Under such situations where information security and individual authentication which are strong or secure are required, the technology which has high value in construction of more strong or reliable security system which is above use of password and/or various encryption technologies in recent years is Biometrics Authentication. The Biometrics primarily means biometrology, and is directed to scientific field for measuring bio-features of living thing, and there is proposed a technology in which bio-features of the human being are caused to be marker for individual discrimination to numerically express those features to collate them with registered data to thereby perform authentication of the person himself. As well known Biometrics Authentication, there is mentioned a method in which Fingerprint is caused to be authentication marker. For example, in the patent literature 1, there is proposed a technology of detecting fingerprint of user by authentication device provided at mouse. In addition to the above, bioindices such as Ear Scanner, Iris Scanning, Retinal Scanner, Speaker Verification, Palm Print or Venous Pattern, etc. may be used to perform authentication.

In general, sensor for detection of bioindex (hereinafter simply referred to as bioindex sensor) for Biometrics authentication is not required to detect biomarker at all times, but may be adapted so that detection operation can be executed at the time of starting of equipment or at the time of release of security lock. For this reason, even if sensor for fingerprint authentication is prepared (provided) at a position different from the position with which finger of user comes into contact in ordinary state, there is no problem. In actual mobile personal computer or mobile telephone in which fingerprint authentication unit is mounted, such fingerprint authentication sensor is provided at a position which is not obstacle to ordinary use rather than the position with which the finger comes into contact.

However, it is conceivable that the Biometrics technology is not only applied to authentication use purpose, but also is applied/developed with respect to various use purposes in future. As bioindex, there are mentioned, in addition to the above-described example, GSR (Galvanic Skin Reflex), Galvanic Skin Response, pulse wave, and/or body temperature, etc. In this case, not only there exist bioindices explicitly and temporarily detected as in the case of authentication use purpose, but there may be also indices required to be regularly or successively acquired at the time of ordinary use. For example, in the case of utilizing information such as Galvanic Skin Reflect, Galvanic Skin Response, pulse wave and/or body temperature, etc. of user, there was the problem that grasping portion (manner of holding) of user is changed, strength of grasp differs (varies), or a portion is sweated in the middle of measurement so that reliability and stability of values to be measured become bad.

WO-A1-O1/76220, on which the preamble portions of the independent claims are based, discloses a telephone provided with sensors on the handset for body composition analysis, electrocardiogram and pulse measurement function.

An object of the present invention is to provide a novel input device, a novel input method and a novel electronic equipment which are adapted for eliminating problems that prior arts as described above have to thereby have ability to detect bio-information of user.

Another object of the present invention is to provide an input device, an input method and an electronic equipment in which if user executes use by ordinary way of use, bioindices are successfullly acquired even if explicit acquiring operation is not performed, and detection can be stably performed in the state where physiological index to be detected does not change depending upon difference of manner of holding or grasping force, etc. so that reliability of detection value is permitted to be enhanced.

The present invention therefore provides an input device, input method and electronic equipment as defined in the appended claims. The invention is defined in the claims.

In accordance with the input device, the input method and the electronic equipment according to the present invention, it is possible to successively acquire bioindices through skin surface of finger in contact with equipment when user grasps, by finger, the body to be operated for the purpose of performing operation without allowing user to explicitly execute acquiring operation of bioindex. Moreover, when user executes use by way of ordinary use, even if explicit acquiring operation is not performed, bioindices can be successively acquired, and detection can be stably performed in the state where physiological index detected is not changed depending upon difference of manner of holding or grasping force, etc. so that reliability of detection value can be enhanced. If various bioindices can be stably acquired with high reliability, new entertainment use purpose and/or new technical use purpose in which new biometrics technology of bioindex is applied can be created.

The invention will be further described by way of example with reference to the attached drawings, in which:
FIG 1 is a front view showing the state where user grasps non-foldable type mobile telephone by hand.
FIG 2 is a front view showing the state where user grasps foldable type mobile telephone by finger.
FIG 3 is a perspective view showing an example where sensors for detection of GSR (hereinafter simply referred to as GSR sensors) are attached to the side surface portions of the outer peripheral surface side of the non-foldable type mobile telephone.
FIG 4 is a perspective view showing an example where GSR sensors are attached to corner portions of the outer peripheral surface side of the non-foldable type mobile telephone.
FIG 5 is a perspective view showing an example where GSR sensors are attached to operation input buttons of the non-foldable type mobile telephone.
FIG 6 is a view for explaining an example where GSR sensors are attached to the side surface portions of the outer peripheral surface side of the foldable type mobile telephone.
FIG 7 is a perspective view showing an example where GSR sensors are attached to the corner portions of the outer peripheral surface side of the foldable type mobile telephone.
FIG 8 is a perspective view showing an example where GSR sensors are attached on operation input buttons of the foldable type mobile telephone.
FIG 9 is a perspective view showing a mobile telephone in which sensor portion for detection of pulse wave (hereinafter simply refereed to as pulse wave sensor portion) is provided.
FIG 10A is a cross sectional view showing the pulse wave sensor portion attached to the mobile telephone in the state cut in length side direction of the mobile telephone, and FIG 10B is a cross sectional view showing pulse wave sensor portion in the state cut in short side direction of the mobile telephone.
FIG 11 is a perspective view showing the state where user grasps mobile telephone having pulse wave sensor.
FIG 12 is a side view showing pulse wave sensor portion.
FIG 13 is a block diagram showing a bioindex detecting apparatus used in the present invention.
FIG 14 is a flowchart for explaining the processing when the bioindex detecting apparatus judges reliability of detection values at respective biosensors to select an optimum value.

### (1) First embodiment

Initially, the first embodiment of the present invention will be explained. In this embodiment, sensor is provided at a position where if user executes use by ordinary way of use, bioindices can be successively acquired even when explicit acquiring operation is not performed. As bioindex to be detected in this embodiment, there may be bioindex such that an electronic equipment which is a body to be operated is grasped by finger so that bioindex can be detected through skin surface. In this example, as index used in use purpose different from authentication use purpose in which operation for detection is explicitly and temporarily performed, and index required to be successively acquired at the time of ordinary use by user, there are mentioned sweating, heartbeat, pulse wave, skin temperature, Galvanic Skin Reflex, Galvanic Skin Response, MV (Micro Vibration), myoelectric potential, and SP02 (blood oxygen saturation level), etc.

This embodiment is applied to portable or mobile type telephone (hereinafter referred to as mobile telephone) as an electric equipment, and explanation will be given by taking an example of detecting bioindices from user who utilizes the mobile telephone. The bioindices detected here are bioindices which can be relatively easily acquired, and explanation will be given by taking an example of detecting, e.g., Galvanic Skin Reflex, Galvanic Skin Response, pulse wave or body temperature (skin temperature) of user.

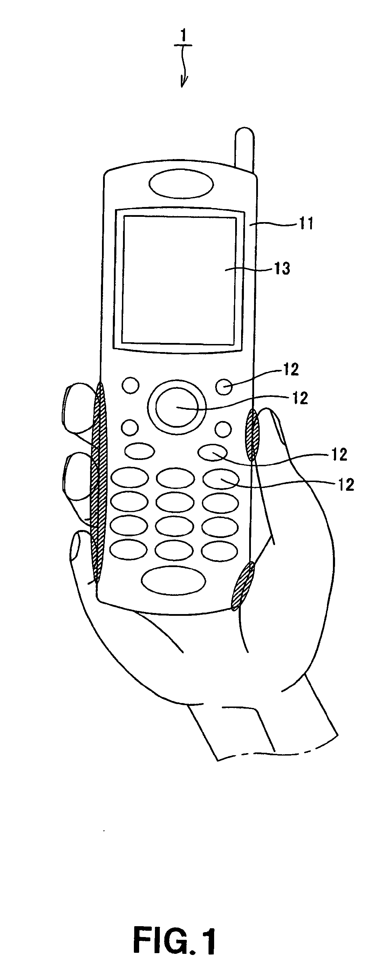

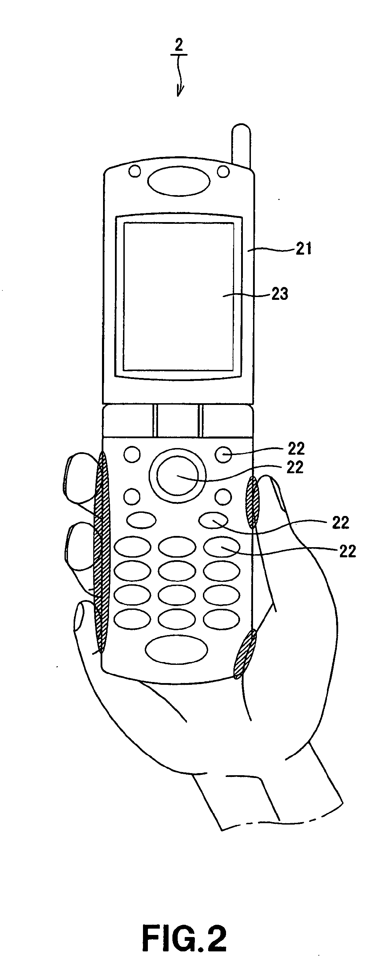

FIGS. 1 and 2 show, in a model form, the state where user grasps the mobile telephone by finger. The regions indicated by slanting lines in the figures are portion with which finger or palm of user come into contact by ordinary use. FIG 1 shows the example of non-foldable type mobile telephone 1, and FIG 2 shows the example of foldable type mobile telephone 2. At front face portions 11, 21 of the outer surfaces of casings constituting the telephone bodies, there are provided operation input buttons 12, 22 and display screens 13, 23 for executing functions of these mobile telephones 1, 2.

Galvanic Skin Reflex or Galvanic Skin Response (GSR) is bioindex used also in the so-called polygraph, and utilizes that electric resistance of skin changes by sweating. In order to measure GSR, it is necessary to detect Galvanic Skin Reflex (Response) between at least two points on the skin. In general, there is employed a technique in which electrode is caused to be in contact with the portion between two points of finger or palm to allow very weak current to flow to detect change quantity of resistance, etc. In view of the above, in the case of the mobile telephone, when the plan surface where display screen which displays guide display and information for operation is provided is caused to be front face portion of the casing as a position where measurement can be successively made even if explicit acquiring operation is not performed when user uses the mobile telephone by ordinary way of use at the time of operation such as calling (talking), and/or mail input, etc., GSR sensor is provided at the side surface portion of the outer peripheral side, the corner portion that the front face portion and the side face portion form, or the surface of the operation input button of the mobile telephone.

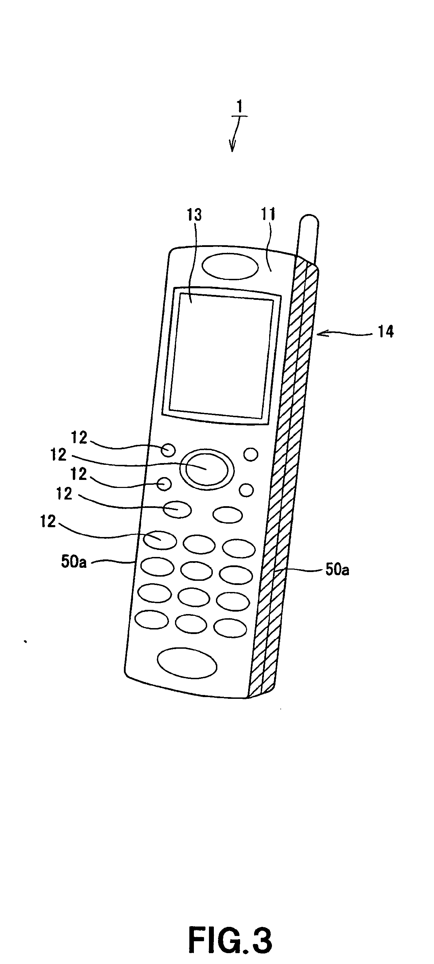

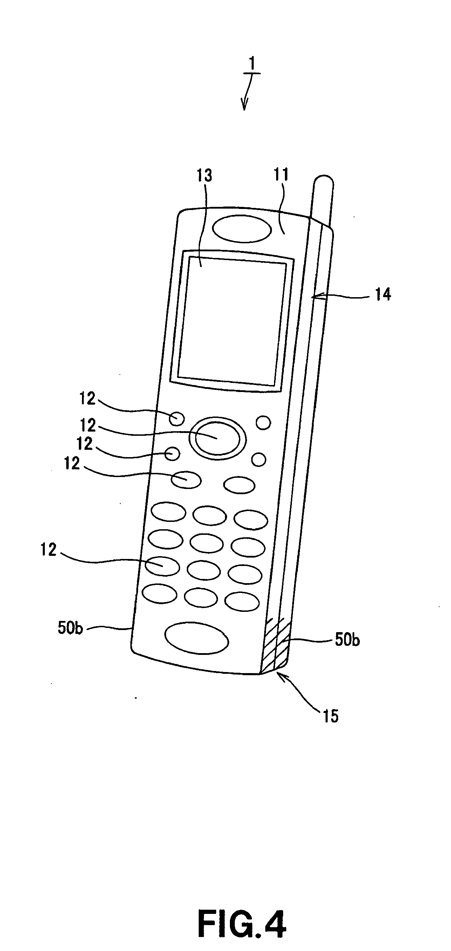

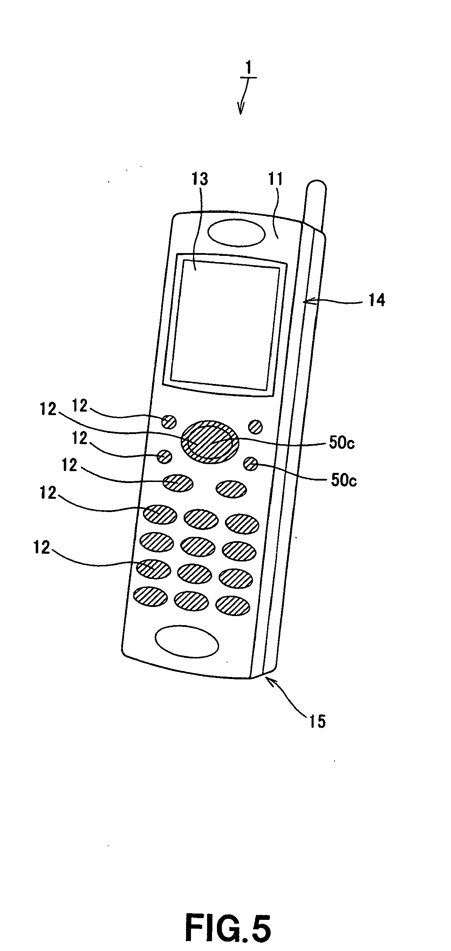

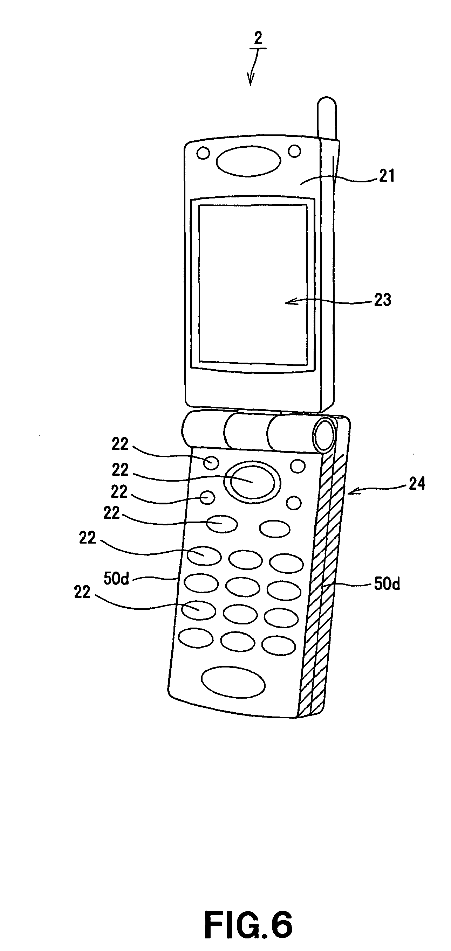

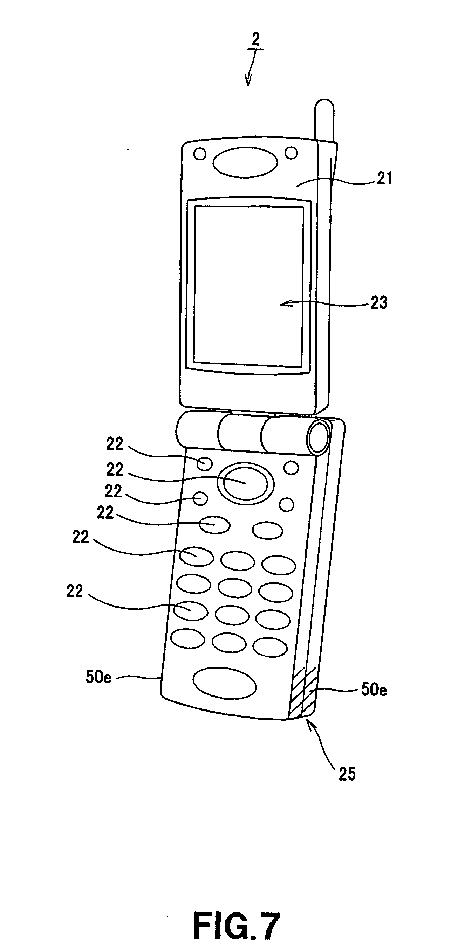

The example where GSR sensors 50a are attached to side surface sides 14 of the outer peripheral side of the non-foldable mobile telephone 1 is shown in FIG 3, the example where GSR sensors 50b are attached to corner portions 15 of the outer peripheral side of the non-foldable type mobile telephone 1 is shown in FIG 4, and the example where GSR sensors 50c are attached on the operation surface with which finger comes into contact of the operation input buttons 12 of the non-foldable type mobile telephone 1 is shown in FIG 5. Moreover, the example where GSR sensors 50d are attached to side surface portions 24 of the outer peripheral sides of the foldable type mobile telephone 2 is shown in FIG. 6, the example where GSR sensors 50e are attached to corner portions 25 of the outer peripheral sides of the foldable type mobile telephone 2 is shown in FIG. 7, and the example where GSR sensors 50f are attached on the operation surfaces where finger comes into contact with operation input buttons 22 of the foldable type mobile telephone 2.

The components indicated by the same reference numerals of the GSR sensors 50 shown in FIGS. 3 to 6 represent a pair of sensors, wherein one sensor is cathode and the other sensor is anode. In the case where the GSR sensor 50 is provided on the operation input button, conductive material is used on the operation input button surface. Moreover, two electrodes spaced by a predetermined distance may be provided on single key so that a pair of sensors are provided.

Moreover, GSR sensors 50 may be provided at both the side surface portions 14 shown in FIG. 3 and the corner portions 15 of the outer peripheral sides of the casing shown in FIG. 4. Further, the GSR sensors 50 may be provided on the operation surfaces of the operation input buttons 12. In this case, e.g., the GSR sensor 50a provided at the side surface portion 14 may be anode, and the GSR sensor 50b provided at the corner portion 15 may be cathode. There may be employed a configuration in which the relationship between the sensor and electrode is opposite to the above. Moreover, GSR sensors 50a, 50b provided at the outer peripheral surface side of the casing are caused to be electrode of one polarity and GSR sensors 50c provided on the operation input buttons disposed on the casing are caused to be electrode of the other polarity, thereby making it possible to detect GSR at the portion between finger by which the operation button is pushed down and palm in contact with the GSR sensor of the outer edge when user pushes down the operation button. It is to be noted that whether electrode provided at an arbitrary portion is anode or cathode can be selected in accordance with optimum measurement distance of GSR.

By providing GSR sensors as described above, when user uses the mobile telephone, it is possible to acquire GSR serving as bioindex by itself by action to grasp such mobile telephone by finger. Moreover, even in the case where user grasps the mobile telephone by any one of right hand and left hand, GSR can be detected.

It is said that the body temperature, particularly the body temperature of the peripheral portion such as finger tip depends upon stress or comfortable/uncomfortable state. For example, when the human being is placed in uncomfortable state generally by stress, circulation of blood of vascular tract of the peripheral portion becomes bad so that temperature (body temperature) is partially lowered. In view of the above, temperature sensors for measuring temperature difference are disposed on the mobile telephone surface to measure temperature difference between the finger tip portion and palm, thereby making it possible to recognize comfortable/uncomfortable state or stress state, etc. of the human being. Similarly to the GSR sensor, temperature sensor may be disposed at the position which has been explained with reference to FIGS. 3 to 6. In the case where the temperature sensors are provided at the non-foldable type mobile telephone 1, these temperature sensors are provided on the side surface portions 14 of the casing, thus to detect finger tip temperature by one sensor, and to detect palm temperature by the other telephone. In addition, one sensor may be provided on the side surface portion 14 of the casing, and the other sensor may be provided at the corner portion of the outer peripheral side of the casing.

In the case where the temperature sensors are provided on the operation surfaces with which finger comes into contact of the operation input buttons, temperature sensor is provided also on the outer peripheral surface of the casing. In this case, fingertip temperature is detected by the sensor provided on the operation input button, and palm temperature is detected by the sensor provided at the casing side. It should be noted that in the case where the temperature sensor is disposed on the outer peripheral surface of the casing, heat insulating processing and/or circuit arrangement within the mobile telephone are changed for the purpose of preventing influence of heat produced from the mobile telephone body.

Subsequently, explanation will be given in connection with the case where mobile telephone is used to detect pulse wave of user as bioindex. It can be considered that pulse wave attempted to detect in this example is equivalent to heartbeat although phase somewhat shifts. In strained or excited state, pulse wave (speed) becomes fast. In stable state, pulse wave (speed) becomes slow. Ordinarily, pulse wave can be detected by optical pulse wave sensor. The optical pulse wave sensor employs a technique of irradiating rays of test light having a specific wavelength from the finger tip nail side to detect rays of transmitted light at the finger tip midsection side. When such a technique is employed, blood oxygen saturation level (SPO2) can be also acquired at the same time. However, in the optical pulse wave sensor, in order to continuously measure stable pulse waves, it is necessary to grasp the mobile telephone in the state where the positional relationship of the finger tip (nail side), the light emitting portion, the finger tip (midsection side) and the light receiving portion is stable to some degree. In view of the above, in mobile telephone 7 which will be explained in this practical example, pulse wave sensor portion comprising pulse wave sensor is provided at a portion of the casing constituting the telephone body.

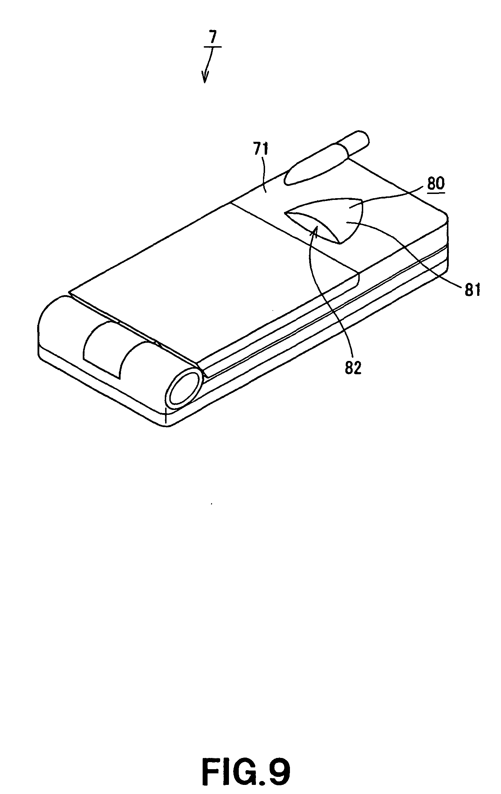

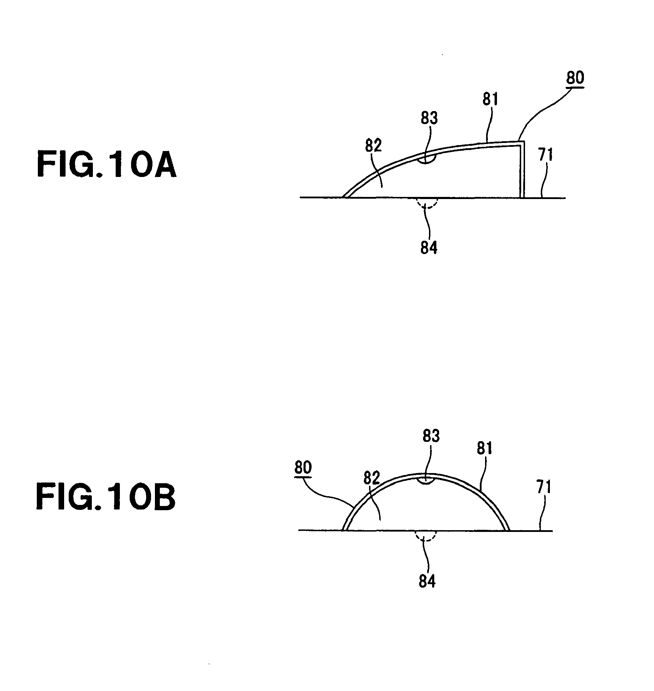

The mobile telephone 7 comprising pulse wave sensor portion 80 is shown in FIG. 9. Moreover, the cross section in which the pulse wave sensor portion 80 is cut in length side direction of the mobile telephone 7 is shown in FIG. 10A, and the cross section in which the pulse wave sensor portion 80 is cut in short side direction of the mobile telephone 7 is shown in FIG. 10B. At the mobile telephone 7, pulse wave sensor portion 80 is provided at a rear face portion 71 of the casing in which display screen serving to display guide display and information for operation is provided on the surface thereof. As shown in FIGS. 10A, 10B, the rear face portion 71 of the casing comprises a finger holding cover 81 having internal shape curved so as to take substantially the same shape as the finger tip shape of user, and a finger tip insertion portion 82 formed between the finger holding cover 81 and the rear face portion 71 to constitute the pulse wave sensor portion 80. The inside surface of the finger holding cover 81 comprises a light emitting portion 83 of the optical pulse wave sensor, wherein a light receiving portion 84 as pulse wave detecting means is provided at the position opposite to the light emitting portion 83 of the rear face portion 71. The light emitting portion 83 is disposed at a position where rays of test light having a specific wavelength can be irradiated substantially onto the finger tip nail upper portion when user inserts finger tip. Rays of test light which have been emitted at the light emitting portion 83 are detected by the light receiving portion 84 disposed at the middle section side of finger when user inserts finger tip as rays of light which have been transmitted through the finger inside.

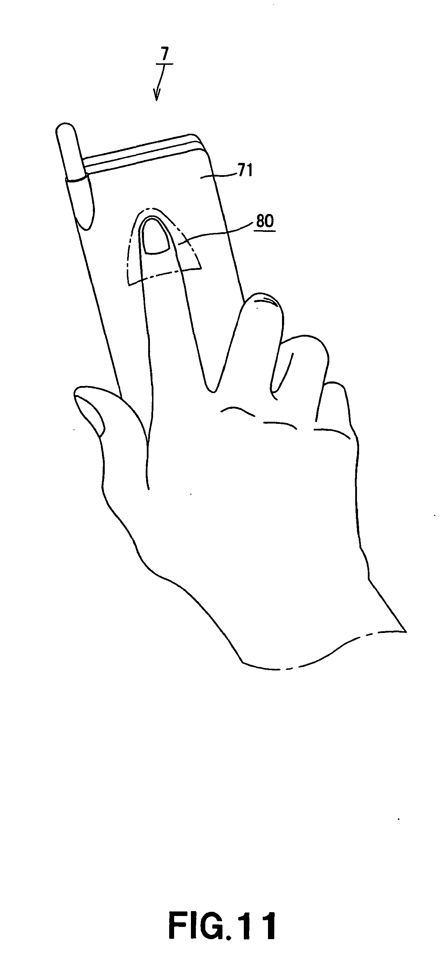

As shown in FIG. 11, if user grasps mobile telephone including pulse wave sensor 80 of such a structure by most popular method, there results the state where when user grasps the mobile telephone 7, the forefinger is inserted into the finger chip insertion portion 82 of the pulse wave sensor portion 80 without disagreement of feeling. Accordingly, for a time period during which user is using the mobile telephone 7, it is possible to perform stable measurement of pulse wave.

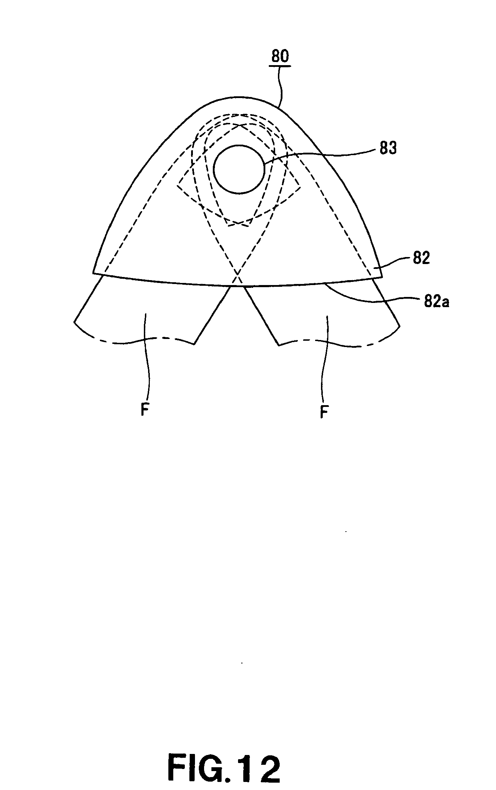

There is individual difference when user uses the mobile telephone depending upon the state where he grasps it by left or right hand. Even in the case of the same person, it is general that way of grasp by finger varies in dependency with calling or mail input. For this reason, as shown in FIG. 12, a finger insertion opening portion 82a of the pulse wave sensor portion 80 is widened in a sector form to devise opening shape of the finger tip insertion portion 82 such that the light emitting portion 83 is positioned substantially at the center of the finger nail even if finger F is inserted at an any angle. Thus, it is possible to comply with, e.g., unevenness of finger tip insertion angle due to difference of hand which grasps the mobile telephone 7. Moreover, the finger holding cover 81 forms curved surface and takes a shape such that the diameter is gradually reduced according as distance from the finger insertion opening portion toward long side direction is increased. For this reason, the degree of reduction of diameter is lowered to thereby broaden the space of the finger tip insertion portion 82. Thus, it is possible to absorb individual difference of size of finger.

In regard to the point where the pulse wave sensor portion 80 is provided so that the rear face portion 71 of the finger holding cover 81 is swelled out in a manner projected therefrom, the portion in which, e.g., the light receiving portion 83 is disposed is allowed to be recessed portion caused to be in conformity with finger shape to thereby suppress projection height from the rear face portion of the finger holding cover 81 to also have ability to realize thin structure.

It is to be noted that the mobile telephone 7 in which pulse wave sensor portion 80 is provided may be non-foldable mobile telephone, or may be foldable mobile telephone.

### (2) Second embodiment

When biosensor for acquiring bioindex of user is attached to an input equipment that user operates in the state where he directly grasps it such as mobile telephone, remote controller, controller of television game machine, and/or mouse used as input means of computer, etc., measurement of bioindex which is tacit and is non-invasive/assaultive to user can be made. Particularly, since heartbeat, pulse wave, SPO2 (blood oxygen saturation level), skin temperature, Galvanic Skin Reflex and/or Galvanic Skin Response are bioindex which can be detected through the skin surface, even if operation for detection is not performed explicitly and temporarily, user grasps it by finger in use so that such bioindex can be acquired. If sensor position is devised, measurement can be made if ordinary use is performed without necessity of changing manner of holding or grasping the device. As bioindex which can be detected through skin surface, there are mentioned sweating, MV (Micro Vibration) and/or myoelectric potential, etc.

In the second embodiment, there are proposed a device and a method in which plural sensors are provided at an equipment utilizing bioindex of user to thereby have ability to detect bioindex data stably and with high accuracy. Initially, whether any data is used among bioindex data which have been acquired by the plural sensors is determined.

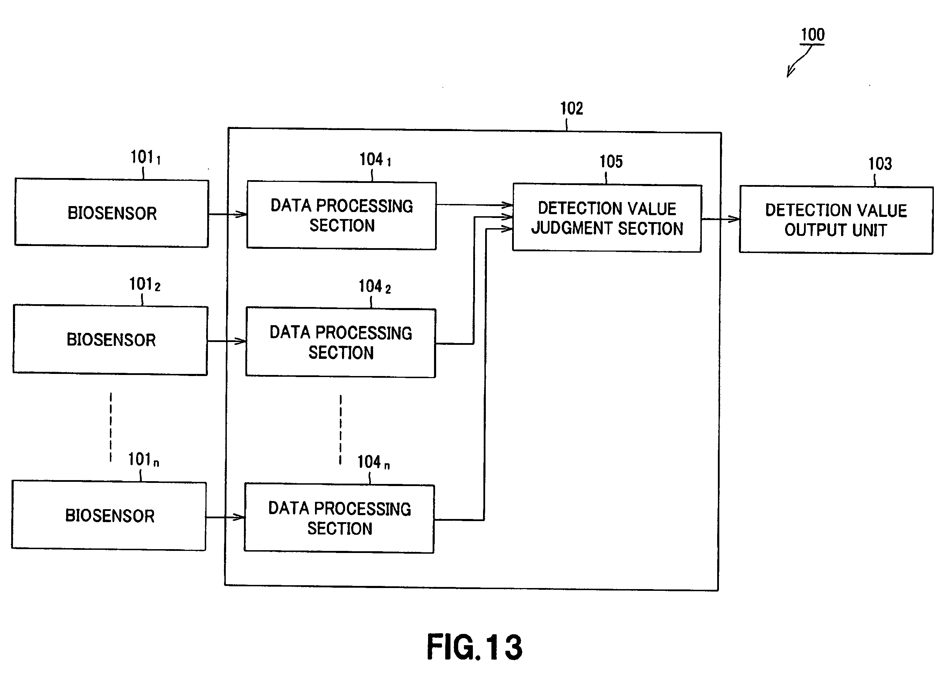

The fundamental configuration of a bioindex detecting apparatus of this embodiment will be explained by using FIG. 13. There is employed an approach to select stable value and value having high reliability among output values of biosensors disposed at plural portions, or to perform integrating processing of these values, thus to realize reliable data detection. Electronic equipment to be attached and attachment position thereof will be described later.

As shown in FIG. 13, the bioindex detecting apparatus 100 comprises biosensors 101₁, 101₂, ..., 101ₙ, a detection value selector unit 102 for selecting any one of detection values which have been detected by these biosensors to output the selected detection value, and a detection value output unit 103 for outputting a detection value which has been selected by the detection value selector unit 102. Further, the detection value selector unit 102 comprises data processing sections 104₁, 104₂, ..., 104ₙ for processing detection values which have been detected by the respective biosensors, and a detection value judgment section 105 for selecting an optimum value from detection values which have been processed by the data processing sections, wherein respective sections are caused to undergo supervisory control by control section (not shown). Controls of the respective sections may be performed by the control section of electronic equipment in which the bioindex detecting apparatus 100 is provided.

From the detection value output unit 103, a detected bioindex is sent to the electronic equipment in which the bioindex detecting apparatus 100 is provided. The outputted bioindex is used at respective electronic equipments. There may be employed use purpose such that, e.g., if the electronic equipment is mobile telephone, the detected bioindex of user is transmitted to the opposite side of communication, and if the electronic equipment is remote controller of the air conditioner, temperature setting is made in accordance with the detected bioindex of user, or the like.

The biosensor 101 is a sensor for detecting bioindex which can be detected by contact of the skin surface, and a sensor for heartbeat, pulse wave, SPO2 (blood oxygen saturation level), skin temperature, Galvanic Skin Reflex or Galvanic Skin Response may be applied for this purpose. Plural sensors selected therefrom are provided on an electronic equipment surface such that bioindex can be acquired even if user does not change manner of holding again when user grasps the electronic equipment by finger in ordinary use, or finger comes into contact therewith. Moreover, while the biosensors 101 are respectively the same sensor in the example shown in FIG. 13, there may be employed different kinds of biosensors for detecting the same bioindex by different techniques, or different bioindices may be detected by different kinds of biosensors.

In this example, the data processing sections 104 in the detection value selector unit 102 calculate SN (signal-to-noise) ratios of respective output values of bioindex which have been detected by the biosensors. The SN ratios which have been calculated with respect to output values from the respective sensors are sent to the detection value judgment section 105. The data processing sections 104 may be processing sections for calculating detection levels of detected output values or auto-correlation functions of detected output values also in addition to the SN ratio. In the case where respective plural biosensors 101 are the same biosensor for detecting the same bioindex, the detection value judgment section 105 may select, as output value, value which has been detected substantially as the same value by plural sensors, or may calculate average value of values detected by respective biosensors to select the average value thus calculated as an output value. In addition, in the case where detection value is caused to undergo digital processing, error rates may be compared to select detection value having small error.

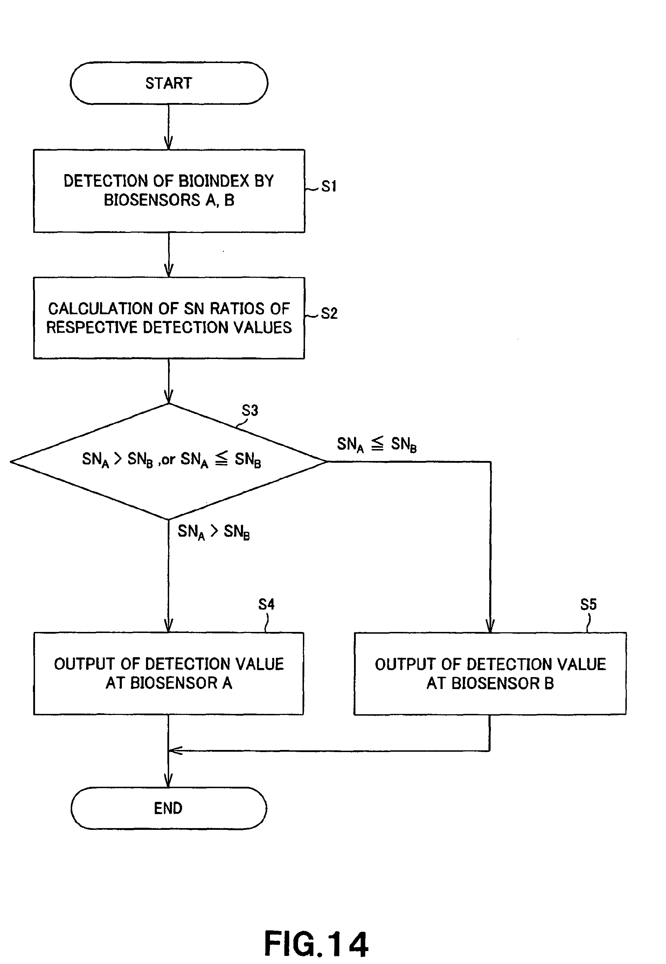

Subsequently, the processing in which the bioindex detecting apparatus judges reliability of detection values at respective biosensors to select an optimum value is shown in FIG. 14. FIG. 14 shows the processing for selecting detection values which have been detected by two biosensors of the biosensor A and the biosensor B. The biosensor A and the biosensor B are the same biosensor for detecting the same bioindex.

As step S1, the biosensor A and the biosensor B detect bioindex values. The detection values of the bioindex are caused to undergo data processing at step S2 so that SN ratios are determined. Then, at step S3, SN ratios are compared with each other. Here, whether or not any one of SN ratio of detection value at the biosensor A (SNA) and SN ratio of detection value at the biosensor B (SNB) is high is discriminated. Namely, if SNA>SNB, detection value at the biosensor A is selected. At step S4, value which has been detected at the biosensor A is sent to the detection value output section. On the other hand, if SNA≤SNB, detection value at the biosensor B is selected. At step S5, value which has been detected at the biosensor B is sent to the detection value output unit.

At the step S4 of FIG 14, data of the biosensor A having SN higher than that of the biosensor B is outputted. Since data having high SN ratio is selected at all times in accordance with this processing, biosensors to be selected change without interruption in a manner of, e.g., A→B→A→A→B with time change. It is considered that the processing shown in FIG. 14 is effective particularly in the case where one detection value is obtained by one sensor, e.g., in the case where body temperature (finger tip temperature and palm temperature) is detected, etc.

Moreover, as another example, in the case where threshold value is provided at detection value so that the biosensors A and B both have not a predetermined SN or more, output is not provided. In this case, if that threshold value is caused to be SNSH, SNA>SNB≥SNSH or SNB>SNA≥SNSH is judged at the step S3.

Furthermore, in the case where threshold value is provided in difference between detection values of both biosensors so that unevenness between detection values of the both biosensors is large, output may not be provided. In this case, at the step S3, whether |SNA-SNB|≥SNSH and SNA>SNB, or |SNA-SNB|≥SNSH and SNB≥SNA is judged.

As practical example of the embodiment, the state where mobile telephone is used to detect bioindex of user who utilizes the mobile telephone will be described below. The bioindex detected here is bioindex which can be relatively easily acquired from user who uses mobile telephone, and there are mentioned, e.g., Galvanic Skin Reflex, Galvanic Skin Response, pulse wave and/or body temperature (skin temperature) of user.

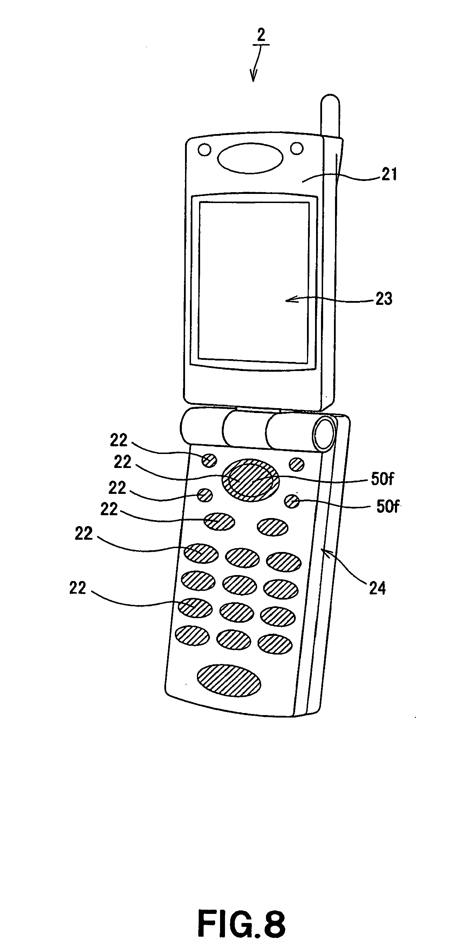

It is to be noted that since practical example of attachment with respect to the mobile telephone of GSR sensor 50 is similar to that explained by using FIGS. 3 to 8 in the first embodiment, the FIGS. 3 to 8 are referred to thereby omit further detailed explanation.

Initially, for the purpose of determining GSR, it is necessary to detect Galvanic Skin Reflect (Response) between at least two points on the skin. While there is generally employed a technique in which electrode is caused to be in contact with the portion between two points of finger or palm to allow very weak current to flow to detect change quantity of resistance, etc., plural pairs of sensors each comprised of a pair of electrodes are provided in this example to select output from an optimum sensor pair.

Respective GSR sensors 50a, 50b, 50c, 50d, 50e, 50f provided on the outer peripheral surface of the side surface portion, etc. of the casing constituting the telephone body, and or the operation surface of the operation input button thereof are divided into plural regions to constitute respective one sensors with respective region pairs being as one set. In the examples shown in FIGS. 3 to 8, respective sets of sensors are constituted by pairs of GSR sensors 50a, 50b, 50c, 50d, 50e, 50f indicated by the same reference numerals.

Here, one of each paired GSR sensors 50a, 50b, 50c, 50d, 50e, 50f constitutes cathode, and the other GSR sensor constitutes anode.

In this example, sensor provided on the operation surface with which finger comes into contact of the operation input button is constituted by depositing conductive material on the surface of the operation input button. In addition, two electrodes may be provided in the state spaced by a predetermined distance on one operation input button to thereby a pair of GSR sensors.

By providing the GSR sensors 50a, 50b, 50c, 50d, 50e, 50f in a manner as shown in the previously mentioned FIGS. 3 to 8, when user uses the mobile telephone, GSR serving as bioindex can be acquired by itself by the action to grasp the mobile telephone 1 or 2 as previously described. Moreover, by providing the GSR sensors 50a, 50b, 50c, 50d, 50e, 50f in a manner as shown in FIGS. 3 to 8, even in the case where user grasps the mobile telephone 1 or 2 by any one of right and left hands, it is possible to perform detection of GSR.

Similarly to the GSR sensors, temperature sensors may be disposed at the positions which have been explained with reference to FIGS. 3 to 6. In the case where the temperature sensors are provided at the non-foldable type mobile telephone 1, there is employed, similarly to the GSR sensor, an approach in which plural temperature sensors are provided at the side surface portions 14 of the casing to detect temperature of finger tip by one group of sensors, and to detect temperature of palm by the other group of sensors. Moreover, there may be also employed a configuration in which one group of sensors are provided at the side surface portions 14 of the casing, and the other group of sensors are provided at the corner portions of the outer peripheral side of the casing.

In the case where temperature sensors are provided on the operation input button, one of the pair of sensors is provided at the operation input button side, and the other sensor is provided at the outer peripheral surface side of the casing to constitute a set of temperature sensors. In this case, finger tip temperature is detected by the sensor provided on the operation surface of the operation input button, and palm temperature is detected by the sensor provided at the casing side. It is to be noted that in the case where the temperature sensor is disposed on the surface of the casing, heat insulating processing and/or circuit arrangement within the mobile telephone are changed for the purpose of preventing influence of heat produced from the mobile telephone body.

Also in the case where the mobile telephone is used to detect pulse wave of user as bioindex, pulse wave sensor similar to the first embodiment is used.

While pulse wave sensors may be provided at plural portions in this practical example, there may be also employed a configuration, as has been explained with reference to FIGS. 9 to 12, in which single pulse wave sensor is provided, and GSR sensor or temperature sensor may be further provided.

As explained also in the above-described first embodiment, plural biosensors may be the same kind of sensors, or may be different kind of biosensors also in this embodiment.

As described above, biosensors are provided at plural portions of the surface of the electronic equipment such as mobile telephone, etc. to acquire bioindex values from the plural portions to thereby eliminate unevenness of detection values taking place by difference of manner of holding or grasping of the electronic equipment or difference of the state of skin surface, etc. thus to have ability to improve reliability of detection values. Moreover, there are some mobile telephones capable of performing calling operation (talking) as telephone and further having mail transmit/receive function. In such a case, it is also possible to comply with difference of manner holding or grasping in use as in the case of the time of calling and the time of preparing mail. Further, in future, it is predicted that there will appear electronic equipments utilizing biometrics in the field except for authentication. It cannot be said that the bioindices are all directed to bioindices in which input operation (authentication operation) is explicitly and temporarily performed as in the case of fingerprint authentication, for example. In accordance with this embodiment, it is possible to acquire bioindex more simply and stably. Thus, it is possible to improve reliability of bioindex values acquired at the biometrics application equipments.

It is to be noted that while the invention has been described in accordance with certain preferred embodiments thereof illustrated in the accompanying drawings and described in the above description in detail, it should be understood by those ordinarily skilled in the art that the invention is not limited to the embodiments, but various modifications, alternative constructions or equivalents can be implemented without departing from the scope of the present invention as set forth and defined by the appended claims.

### Industrial Applicability

While the present invention has been explained by taking an example of mobile telephone as portable or mobile electronic equipment, the present invention can be applied, without being limited to mobile telephone, also to any input devices comprising input unit such as operation button, etc. which are used in the state grasped by finger and serving to perform input of operation instructions and/or various information. For example, in recent years, digital televisions of the interactive (bidirectional) communication system, etc. have appeared. Bioindex detecting means may be disposed, in the above-described manner, at remote controller of multi-functional television and/or audio-visual equipment, and/or controller of television game machine, etc. Moreover, bioindex detecting means is provided within a region including position with which finger comes into contact of the surface of body to be operated that user grasps by finger in use such as control (steering) lever of ship or airplane, etc. and/or handle of automotive vehicle, etc. to thereby have ability to successively acquire bioindices through skin surface from contact when user grasps the body to be operated for the purpose of performing operation without allowing user to explicitly execute acquiring operation of bioindex. In addition, if various bioindices can be acquired, there can be created new entertainment use purpose and/or new technical use purpose in which biometrics technology of new bioindies is applied. For example, the present invention can be applied to information communication equipments in which a detected bioindex of user is transmitted to the opposite side of communication, or information output corresponding to biostate calculated from bioindex of user is provided, and/or life equipments such that temperature setting is made in accordance with detected bioindex of user, etc.

## Claims

1. An input device (1) comprising: bioindex detecting means (101) provided within a region including a holding position for the surface of a body to be operated, that user holds in use, and for detecting, for a time period during which user grasps the body to be operated, bioindex of the user through skin of the user, the bioindex being at least one of sweating, heartbeat, pulse wave, Galvanic Skin Reflex, Galvanic Skin Response, Micro Vibration, myoelectric potential, blood oxygen saturation level, and body temperature, and combination of these bioindices; and bioindex analyzing means (102) for analyzing the bioindex which has been detected by the bioindex detecting means (101); **characterized in that**:
plural different kinds of bioindex detecting means (101) for detecting the same bioindex by different techniques are provided, and by selector means (105) for selecting at least one bioindex information from bioindex information which have been detected by the plural bioindex detecting means (101), the bioindex analyzing means (102) serving to analyze the bioindex information which has been selected by the selector means (105).

2. The input device (1) according to claim 1, wherein the bioindex detecting means (101) is detecting means for detecting Galvanic Skin Reflex or Galvanic Skin Response between predetermined two points of palm of one hand of user.

3. The input device (1) according to claim 1, wherein the bioindex detecting means (101) is pulse wave detecting means for detecting pulse wave of user.

4. The input device (1) according to claim 1, wherein the bioindex detecting means (101) is temperature detecting means for detecting body temperature of user.

5. The input device (1) according to claim 4, wherein the temperature detecting means is composed of finger tip temperature detecting means for detecting finger tip temperature provided at a position with which finger tip comes into contact when the finger tip temperature detecting means is grasped by finger of the user, and palm temperature detecting means provided at a position with which palm of the user comes into contact and for detecting palm temperature.

6. The input device (1) according to claim 1, wherein the selector means (105) serves to compare signal-to-noise ratios of output values which have been detected by the plural bioindex detecting means (101) to select an output value having value of higher signal-to-noise ratio.

7. The input device (1) according to claim 1, wherein the selector means (105) serves to compare detection levels of output values which have been detected by the plural bioindex detecting means (101) to select an output value having higher detection level.

8. The input device (1) according to claim 1, wherein the selector means (105) serves to compare auto-correlation functions of output values which have been detected by the plural bioindex detecting means (101) to select an output value in which correlation has been taken to more degree.

9. The input device (1) according to claim 1, wherein the selector means (105) serves to select one output from outputs from the plural bioindex detecting means (101).

10. The input device (1) according to claim 1, wherein the selector means (105) serves to select, as an output value, a value which has been detected substantially as the same value at the plural bioindex detecting means (101).

11. The input device (1) according to claim 1, wherein the selector means (105) serves to select, as an output value, an average value obtained by averaging values detected at the respective bioindex detecting means (101).

12. The input device (1) according to claim 1, wherein the respective plural bioindex detecting means (101) further comprise different kinds of bioindex detecting means (101) for detecting different bio indices.

13. The input device (1) according to claim 1, wherein the input device (1) is provided at an operation input unit of any one of electronic equipments including personal computer, television image receiver, video and/or audio signal recording and/or reproducing device and air conditioner.

14. The input device (1) according to claim 1, wherein the input device (1) is provided at a controller for television game machine.

15. The input device (1) according to claim 1, wherein each of the plural bioindex detecting means (101) is provided at a control or steering unit that user holds in control or steering at any one of machines to be controlled including automotive vehicle, train, airplane, ship and industrial machinery.

16. An input method including:
a bio index detection step (S1) of detecting, by detecting means (101) provided within a region including a holding position for the surface of a body to be operated, that user holds in use, bioindex of the user through skin of the user for a time period during which the user holds the body to be operated, the bioindex being at least one of sweating, heartbeat, pulse wave, Galvanic Skin Reflex, Galvanic Skin Response, Micro Vibration, myoelectric potential, blood oxygen saturation level, and body temperature, and combination of these bioindices; and a bioindex analysis step of analyzing bioindex which has been detected at the bioindex detection step (S1); **characterised in that**:
the bioindex detection step consists of plural bioindex detection steps detecting the same bioindex by different techniques, the input method including: a selection step (S3) of selecting at least one bioindex information from bioindex information which have been detected at the plural bioindex detection steps; and a bioindex analysis step of analyzing bioindex information which has been selected at the selection step (S3).

17. The input method according to claim 16, further comprising plural bioindex detection steps which detect different bioindices.

18. An electronic equipment (1) including an input device according to any one of claims 1 to 15, said region including a holding position for the surface of a body to be operated, with which finger comes into contact when user grasps the body to be operated through the finger in use.

19. The electronic equipment (1) according to claim 18, wherein display means (13) for displaying guide display for operation and information is provided at the front face portion of a casing (11), the detecting means being provided at the side surface portion (14) of the casing (11).

20. The electronic equipment (1) according to claim 19, comprising: operation means (12) for an operation input, wherein the detecting means is provided at a position with which finger of user comes into contact of the surface of the operation means (12).

21. The electronic equipment (1) according to claim 19, wherein the detecting means is provided at the corner portion of the casing (11).

22. The electronic equipment (1) according to claim 18, wherein display means (13) for displaying guide display for operation and information is provided at the front face portion of the casing (11), and pulse wave detecting means as said bioindex detecting means (101) is provided at the rear face portion opposite to the front face portion of the casing (11).

23. The electronic equipment (1) according to claim 22, wherein a detection portion (80) comprising a finger holding cover (81) having internal surface shape curved so as to take substantially the same shape as finger tip shape of the user, and a finger chip insertion portion (82) formed between the finger holding cover (81) and the rear face of the casing (11) is provided at the rear face portion side of the casing (11), light emitting means (83) being provided at the inner surface of the finger holding cover (81), light receiving means (84) as the pulse wave detecting means being provided at the rear face of the casing (11) opposite to the light emitting means (83).

24. The electronic equipment (1) according to claim 18, comprising: display means (13) serving to display guide display for operation and information at an outer casing front face portion (11), wherein one of temperature detecting means is provided as said bioindex detecting means (101) at the side surface portion with respect to the outer casing front face portion (11).

25. The electronic equipment (1) according to claim 18, comprising: operation means (12), wherein finger tip temperature detecting means as said bioindex detecting means (101) is provided at a position with which finger of user comes into contact of the surface of the operation means (12).

26. The electronic equipment (1) according to claim 18, wherein the palm temperature detecting means is provided at the corner portion of the outer peripheral surface side of the casing (11).

27. The electronic equipment (1) according to claim 18, wherein a detecting portion (80) comprising a finger holding cover (81) having an internal surface shape curved so as to take substantially the same shape as finger tip shape of the user, and a finger tip insertion portion (82) formed between the finger holding cover (81) and the rear face of the casing (11) is provided at the rear face portion side of the casing, finger tip temperature detecting means as said bioindex detecting means (101) being provided at the rear face portion of the casing (11).

## Patentansprüche

1. Eingabeeinrichtung (1), die Folgendes umfasst:
Bioindexdetektierungsmittel (101), in einem Gebiet vorgesehen, das eine Halteposition für die Oberfläche eines zu betätigenden Körpers enthält, den ein Benutzer bei Verwendung hält, und zum Detektieren, für eine Zeitperiode, während der der Benutzer den zu betätigenden Körper greift, eines Bioindexes des Benutzers durch die Haut des Benutzers, wobei der Bioindex mindestens einer der Folgenden ist: Schweiß, Herzschlag, Impulswelle, galvanischer Hautreflex, elektrodermale Aktivität, Mikroschwingung, myoelektrisches Potential, Blutsauerstoffsättigung und Körpertemperatur und Kombinationen dieser Bioindizes; und Bioindexanalysierungsmittel (102) zum Analysieren des Bioindexes, der durch das Bioindexdetektierungsmittel (101) detektiert worden ist;
**dadurch gekennzeichnet, dass**:
mehrere verschiedene Arten von Bioindexdetektierungsmitteln (101) zum Detektieren des gleichen Bioindexes durch unterschiedliche Techniken vorgesehen werden und durch Wählmittel (105) zum Wählen mindestens einer Bioindexinformation aus Bioindexinformationen, die durch die mehreren Bioindexdetektierungsmittel (101) detektiert worden sind, wobei die Bioindexanalysierungsmittel (102) zum Analysieren der Bioindexinformationen, die durch das Wählmittel (105) gewählt worden sind, dienen.

2. Eingabeeinrichtung (1) nach Anspruch 1, wobei das Bioindexdetektierungsmittel (101) ein Detektierungsmittel zum Detektieren eines galvanischen Hautreflexes oder einer elektrodermalen Aktivität zwischen zwei vorbestimmten Punkten einer Handfläche einer Hand eines Benutzers.

3. Eingabeeinrichtung (1) nach Anspruch 1, wobei das Bioindexdetektierungsmittel (101) ein Impulswellendetektierungsmittel zum Detektieren einer Impulswelle des Benutzers ist.

4. Eingabeeinrichtung (1) nach Anspruch 1, wobei das Bioindexdetektierungsmittel (101) ein Temperaturdetektierungsmittel zum Detektieren der Körpertemperatur des Benutzers ist.

5. Eingabeeinrichtung (1) nach Anspruch 4, wobei das Temperaturdetektierungsmittel aus einem Fingerspitzentemperaturdetektierungsmittel zum Detektieren einer Fingerspitzentemperatur ist, das an einer Position vorgesehen ist, mit der eine Fingerspitze in Kontakt kommt, wenn das Fingerspitzentemperaturdetektierungsmittel von einem Finger des Benutzers ergriffen wird, und Handflächentemperaturdetektierungsmittel, das an einer Position vorgesehen ist, mit der eine Handfläche des Benutzers in Kontakt kommt, und zum Detektieren einer Handflächentemperatur.

6. Eingabeeinrichtung (1) nach Anspruch 1, wobei das Wählermittel (105) zum Vergleichen von Signal-Rausch-Verhältnissen von Ausgabewerten dient, die von den mehreren Bioindexdetektierungsmitteln (101) detektiert worden sind, um einen Ausgabewert mit einem Wert mit einem höheren Signal-Rausch-Verhältnis zu wählen.

7. Eingabeeinrichtung (1) nach Anspruch 1, wobei das Wählermittel (105) zum Vergleichen von Detektionsgraden von Ausgabewerten dient, die durch die mehreren Bioindexdetektierungsmittel (101) detektiert worden sind, um einen Ausgabewert mit einem höheren Detektionsgrad zu wählen.

8. Eingabeeinrichtung (1) nach Anspruch 1, wobei das Wählermittel (105) zum Vergleichen von Autokorrelationsfunktionen von Ausgabewerten dient, die von den mehreren Bioindexdetektierungsmitteln (101) detektiert worden sind, um einen Ausgabewert zu wählen, bei dem die Korrelation auf mehr Grad genommen worden ist.

9. Eingabeeinrichtung (1) nach Anspruch 1, wobei das Wählermittel (105) zum Wählen einer Ausgabe unter Ausgaben von den mehreren Bioindexdetektierungsmitteln (101) dient.

10. Eingabeeinrichtung (1) nach Anspruch 1, wobei das Wählermittel (105) dazu dient, als einen Ausgabewert einen Wert zu wählen, der im Wesentlichen als der gleiche Wert an den mehreren Bioindexdetektierungsmitteln (101) detektiert worden ist.

11. Eingabeeinrichtung (1) nach Anspruch 1, wobei das Wählermittel (105) dazu dient, als einen Ausgabewert einen Mittelwert zu wählen, der durch Mitteln von an den jeweiligen Bioindexdetektierungsmitteln (101) detektierten Werten erhalten wird.

12. Eingabeeinrichtung (1) nach Anspruch 1, wobei die jeweiligen mehreren Bioindexdetektierungsmittel (101) weiterhin unterschiedliche Arten von Bioindexdetektierungsmitteln (101) zum Detektieren verschiedener Bioindizes umfassen.

13. Eingabeeinrichtung (1) nach Anspruch 1, wobei die Eingabeeinrichtung (1) an einer Betätigungseingabeeinheit eines beliebigen von Elektronikgeräten vorgesehen ist, einschließlich PCs, Fernsehbildempfänger, Video- und/oder Audiosignalaufzeichnungs- und/oder -wiedergabeeinrichtung und Klimaanlage.

14. Eingabeeinrichtung (1) nach Anspruch 1, wobei die Eingabeeinrichtung (1) an einem Controller für eine Fernsehspielmaschine vorgesehen ist.

15. Eingabeeinrichtung (1) nach Anspruch 1, wobei jedes der mehreren Bioindexdetektierungsmittel (101) an einer Steuer- oder Lenkeinheit vorgesehen ist, die ein Benutzer beim Steuern oder Lenken an einer beliebigen von zu steuernden Maschinen hält, einschließlich Kraftfahrzeug, Zug, Flugzeug, Schiff und Industriemaschinen.

16. Eingabeverfahren, das Folgendes beinhaltet:
einen Bioindexdetektierungsschritt (S1) zum Detektieren, durch Detektierungsmittel (101), die in einem Gebiet vorgesehen sind, einschließlich einer Haltposition für die Oberfläche eines zu betätigenden Körpers, den der Benutzer bei Verwendung hält, eines Bioindexes des Benutzers durch die Haut des Benutzers für eine Zeitperiode, während der der Benutzer den zu betätigenden Körper hält, wobei der Bioindex mindestens einer der Folgenden ist: Schweiß, Herzschlag, Impulswelle, galvanischer Hautreflex, elektrodermale Aktivität, Mikroschwingung, myoelektrisches Potential, Blutsauerstoffsättigung und Körpertemperatur und Kombinationen dieser Bioindizes; und einen Bioindexanalyseschritt des Analysierens eines Bioindexes, der bei dem Bioindexdetektierungsschritt (S1) detektiert worden ist; **dadurch gekennzeichnet, dass**
der Bioindexdetektierungsschritt aus mehreren Bioindexdetektierungsschritten besteht, die den gleichen Bioindex durch verschiedene Techniken detektieren, wobei das Eingabeverfahren Folgendes beinhaltet: einen Wählschritt (S3) zum Wählen mindestens einer Bioindexinformation aus Bioindexinformationen, die bei den mehreren Bioindexdetektierungsschritten detektiert worden sind; und einen Bioindexanalyseschritt des Analysierens von Bioindexinformationen, die bei dem Wählschritt (S3) gewählt worden sind.

17. Eingabeverfahren nach Anspruch 16, weiterhin umfassend mehrere Bioindexdetektierungsschritte, die verschiedene Bioindizes detektieren.

18. Elektronikgerät (1) einschließlich einer Eingabeeinrichtung gemäß einem der Ansprüche 1 bis 15, wobei das Gebiet eine Halteposition für die Oberfläche eines zu betätigenden Körpers enthält, mit dem der Finger in Kontakt kommt, wenn der Benutzer bei Benutzung den durch den Finger zu betätigenden Körper ergreift.

19. Elektronikgerät (1) nach Anspruch 18, wobei ein Displaymittel (13) zum Anzeigen eines Führungsdisplays für Betrieb und Informationen an dem Vorderflächenabschnitt eines Gehäuses (11) vorgesehen ist, wobei das Detektierungsmittel an dem Seitenoberflächenabschnitt (14) des Gehäuses (11) vorgesehen ist.

20. Elektronikgerät (1) nach Anspruch 19, das Folgendes umfasst: Betätigungsmittel (12) für eine Betätigungseingabe, wobei das Detektierungsmittel an einer Position vorgesehen ist, mit der ein Finger des Benutzers die Oberfläche des Betätigungsmittels (12) kontaktiert.

21. Elektronikgerät (1) nach Anspruch 19, wobei das Detektierungsmittel an dem Eckabschnitt des Gehäuses (11) vorgesehen ist.

22. Elektronikgerät (1) nach Anspruch 18, wobei ein Displaymittel (13) zum Anzeigen eines Führungsdisplays für Betrieb und Informationen an dem Vorderflächenabschnitt des Gehäuses (11), und ein Impulswellendetektierungsmittel als das Bioindexdetektierungsmittel (101) an einem Rückflächenabschnitt gegenüber dem Vorderflächenabschnitt des Gehäuses (11) vorgesehen ist.

23. Elektronikgerät (1) nach Anspruch 22, wobei ein Detektionsabschnitt (80) eine Fingerhalteabdeckung (81) mit einer inneren Oberflächengestalt umfasst, die so gekrümmt ist, dass sie im Wesentlichen die gleiche Gestalt wie die Fingerspitzengestalt des Benutzers annimmt, und einen Fingerspitzeneinfügeabschnitt (82), der zwischen der Fingerhalteabdeckung (81) und der Rückfläche des Gehäuses (11) ausgebildet ist, auf der Rückflächenabschnittseite des Gehäuses (11) vorgesehen ist, wobei Lichtemittierungsmittel (83) an der inneren Oberfläche der Fingerhalteabdeckung (81) vorgesehen sind, Lichtempfangsmittel (84) als das Impulswellendetektierungsmittel an der Rückfläche des Gehäuses (11) gegenüber dem Lichtemittierungsmittel (83) vorgesehen sind.

24. Elektronikgerät (1) nach Anspruch 18, das Folgendes umfasst: Displaymittel (13), die dazu dienen, ein Führungsdisplay für den Betrieb und Informationen an einem äußeren Gehäusefrontflächenabschnitt (11) anzuzeigen, wobei eines von Temperaturdetektierungsmitteln als das Bioindexdetektierungsmittel (101) an dem Seitenoberflächenabschnitt bezüglich des äußeren Gehäusefrontflächenabschnitts (11) vorgesehen ist.

25. Elektronikgerät (1) nach Anspruch 18, das Folgendes umfasst: Betätigungsmittel (12), wobei ein Fingerspitzentemperaturdetektierungsmittel als das Bioindexdetektierungsmittel (101) an einer Position vorgesehen ist, an der ein Finger des Benutzers die Oberfläche des Betätigungsmittels (12) kontaktiert.

26. Elektronikgerät (1) nach Anspruch 18, wobei das Handflächentemperaturdetektierungsmittel an dem Eckabschnitt der äußeren peripheren Oberflächenseite des Gehäuses (11) vorgesehen ist.

27. Elektronikgerät (1) nach Anspruch 18, wobei ein Detektierungsabschnitt (80) eine Fingerhalteabdeckung (81) mit einer inneren Oberflächengestalt umfasst, die so gekrümmt ist, dass sie im Wesentlichen die gleiche Gestalt wie eine Fingerspitzengestalt des Benutzers annimmt, und einen Fingerspitzeneinfügeabschnitt (82), der zwischen der Fingerhalteabdeckung (81) und der Rückfläche des Gehäuses (11) ausgebildet ist, an der Rückflächenabschnittseite des Gehäuses vorgesehen ist, wobei ein Fingerspitzentemperaturdetektierungsmittel als das Bioindexdetektierungsmittel (101) an dem Rückflächenabschnitt des Gehäuses (11) vorgesehen ist.

## Revendications

1. Dispositif d'entrée (1) comprenant : un moyen de détection d'indicateur biométrique (101) pourvu dans une région comprenant une position de maintien pour la surface d'un corps sur lequel effectuer une mesure, que l'utilisateur maintient en cours d'utilisation, et pour détecter, pendant une période de temps au cours de laquelle l'utilisateur saisit le corps sur lequel effectuer une mesure, un indicateur biométrique de l'utilisateur à travers la peau de l'utilisateur, l'indicateur biométrique étant au moins un indicateur parmi : la transpiration, le rythme cardiaque, le pouls, le réflexe psychogalvanique, la réponse psychogalvanique, des micro-vibrations, le potentiel myoélectrique, le niveau de saturation du sang en oxygène et la température du corps, et toute combinaison de ces indicateurs biométriques ; et un moyen d'analyse d'indicateur biométrique (102) pour analyser l'indicateur biométrique qui a été détecté par le moyen de détection d'indicateur biométrique (101) ; **caractérisé en ce que** :
plusieurs différents types de moyens de détection d'indicateur biométrique (101) pour détecter le même indicateur biométrique par différentes techniques sont pourvus, et un moyen de sélection (105) pour sélectionner au moins une information d'indicateur biométrique à partir des informations d'indicateur biométrique qui ont été détectées par la pluralité de moyens de détection d'indicateur biométrique (101), le moyen d'analyse d'indicateur biométrique (102) servant à analyser l'information d'indicateur biométrique qui a été sélectionnée par le moyen de sélection (105).

2. Dispositif d'entrée (1) selon la revendication 1, dans lequel le moyen de détection d'indicateur biométrique (101) est un moyen de détection pour détecter un réflexe psychogalvanique ou une réponse psychogalvanique entre deux points de la paume de la main d'un utilisateur.

3. Dispositif d'entrée (1) selon la revendication 1, dans lequel le moyen de détection d'indicateur biométrique (101) est un moyen de détection de pouls pour détecter le pouls d'un utilisateur.

4. Dispositif d'entrée (1) selon la revendication 1, dans lequel le moyen de détection d'indicateur biométrique (101) est un moyen de détection de température pour détecter la température corporelle d'un utilisateur.

5. Dispositif d'entrée (1) selon la revendication 4, dans lequel le moyen de détection de température est composé d'un moyen de détection de la température du bout du doigt destiné à détecter la température du bout du doigt, pourvu à un emplacement avec lequel le bout du doigt vient en contact lorsque le moyen de détection de la température du bout du doigt est saisi par le doigt de l'utilisateur, et un moyen de détection de la température de la paume, pourvu à un emplacement avec lequel la paume de l'utilisateur vient en contact, et qui est destiné à détecter la température de la paume.

6. Dispositif d'entrée (1) selon la revendication 1, dans lequel le moyen de sélection (105) sert à comparer des rapports signal sur bruit de valeurs de sortie qui ont été détectées par plusieurs moyens de détection d'indicateur biométrique (101) pour sélectionner la valeur de sortie ayant la valeur dont le rapport signal sur bruit est le plus élevé.

7. Dispositif d'entrée (1) selon la revendication 1, dans lequel le moyen de sélection (105) sert à comparer des niveaux de détection de valeurs de sortie qui ont été détectées par plusieurs moyens de détection d'indicateur biométrique (101) pour sélectionner la valeur de sortie ayant le niveau de détection le plus élevé.

8. Dispositif d'entrée (1) selon la revendication 1, dans lequel le moyen de sélection (105) sert à comparer des fonctions d'auto-corrélation de valeurs de sortie qui ont été détectées par la pluralité de moyens de détection d'indicateur biométrique (101) pour sélectionner une valeur de sortie pour laquelle la corrélation s'est révélée la plus importante.

9. Dispositif d'entrée (1) selon la revendication 1, dans lequel le moyen de sélection (105) sert à sélectionner une sortie parmi les sorties de la pluralité de moyens de détection d'indicateur biométrique (101).

10. Dispositif d'entrée (1) selon la revendication 1, dans lequel le moyen de sélection (105) sert à sélectionner, comme valeur de sortie, une valeur qui a été détectée sensiblement comme étant la même valeur au niveau de la pluralité de moyens de détection d'indicateur biométrique (101).

11. Dispositif d'entrée (1) selon la revendication 1, dans lequel le moyen de sélection (105) sert à sélectionner, comme valeur de sortie, une valeur moyenne obtenue en moyennant des valeurs détectées au niveau des moyens de détection d'indicateur biométrique (101) respectifs.

12. Dispositif d'entrée (1) selon la revendication 1, dans lequel la pluralité de moyens de détection d'indicateur biométrique (101) comprend en outre différents types de moyens de détection d'indicateur biométrique (101) pour détecter différents indicateurs biométriques.

13. Dispositif d'entrée (1) selon la revendication 1, dans lequel le dispositif d'entrée (1) est pourvu au niveau d'une unité d'entrée de commande de fonctionnement d'un équipement électronique quelconque, dont un ordinateur personnel, un récepteur de télévision, un appareil d'enregistrement et/ou de lecture de signal audio et/ou vidéo, et un climatiseur.

14. Dispositif d'entrée (1) selon la revendication 1, dans lequel le dispositif d'entrée (1) est pourvu au niveau d'un contrôleur d'une machine de jeu pour téléviseur domestique.

15. Dispositif d'entrée (1) selon la revendication 1, dans lequel chacun de la pluralité de moyens de détection d'indicateur biométrique (101) est pourvu au niveau d'une unité de commande ou de direction que l'utilisateur contrôle ou dirige au niveau de l'une quelconque des machines à contrôler, dont un véhicule automobile, un train, un avion, un bateau ou une machine industrielle.

16. Procédé d'entrée comprenant :
une étape de détection d'indicateur biométrique (S1) comprenant le fait de détecter, à l'aide d'un moyen de détection (101) pourvu dans une région comprenant une position de maintien pour la surface d'un corps sur lequel effectuer une mesure, que l'utilisateur maintient en cours d'utilisation, un indicateur biométrique de l'utilisateur à travers la peau de l'utilisateur pendant une période de temps au cours de laquelle l'utilisateur maintient le corps sur lequel effectuer une mesure, l'indicateur biométrique étant au moins un indicateur parmi : la transpiration, le rythme cardiaque, le pouls, le réflexe psychogalvanique, la réponse psychogalvanique, des micro-vibrations, le potentiel myoélectrique, le niveau de saturation du sang en oxygène et la température du corps, et une combinaison de ces indicateurs biométriques ; et une étape d'analyse d'indicateur biométrique de l'indicateur biométrique qui a été détecté lors de l'étape de détection d'indicateur biométrique (S1) ;
**caractérisé en ce que** :
l'étape de détection d'indicateur biométrique consiste en plusieurs étapes de détection d'indicateur biométrique détectant le même indicateur biométrique à l'aide de différentes techniques, le procédé d'entrée comprenant : une étape de sélection (S3) comprenant le fait de sélectionner au moins une information d'indicateur biométrique à partir des informations d'indicateur biométrique qui ont été détectées par la pluralité d'étapes de détection d'indicateur biométrique ; et une étape d'analyse d'indicateur biométrique comprenant le fait d'analyser une information d'indicateur biométrique qui a été sélectionnée lors de l'étape de sélection (S3).

17. Procédé d'entrée selon la revendication 16, comprenant en outre plusieurs étapes de détection d'indicateur biométrique qui détectent différents indicateurs biométriques.

18. Équipement électronique (1) comprenant un dispositif d'entrée selon l'une quelconque des revendications 1 à 15, ladite région comprenant une position de maintien pour la surface d'un corps sur lequel effectuer une mesure, avec lequel un doigt vient en contact lorsqu'un utilisateur saisit le corps sur lequel effectuer une mesure par l'intermédiaire du doigt utilisé.

19. Équipement électronique (1) selon la revendication 18, dans lequel un moyen d'affichage (13) servant à afficher un guide de commande ou d'information est pourvu au niveau de la partie de face avant d'un boîtier (11), le moyen de détection étant pourvu au niveau de la partie de surface latérale (14) du boîtier.

20. Équipement électronique (1) selon la revendication 19, comprenant : un moyen de commande de fonctionnement (12) pour une entrée de commande de fonctionnement, où le moyen de détection est pourvu à un emplacement avec lequel le doigt d'un utilisateur vient au contact de la surface du moyen de commande de fonctionnement (12).

21. Équipement électronique (1) selon la revendication 19, dans lequel le moyen de détection est pourvu au niveau de la partie du coin du boîtier (11).

22. Équipement électronique (1) selon la revendication 18, dans lequel un moyen d'affichage (13) permettant d'afficher un guide de commande ou d'information est pourvu au niveau de la partie de la face avant du boîtier (11), et un moyen de détection de pouls, en tant que dit moyen de détection d'indicateur biométrique (101), est pourvu au niveau de la partie de face arrière opposée à la partie de face avant du boîtier (11).

23. Équipement électronique (1) selon la revendication 22, dans lequel une partie de détection (80) comprenant un couvercle de maintien de doigt (81) ayant une forme de surface interne incurvée de manière à prendre sensiblement la même forme que la forme de l'extrémité du doigt de l'utilisateur, et une partie d'insertion d'extrémité de doigt (82) formée entre le couvercle de maintien de doigt (81) et la face arrière du boîtier (11) est pourvue au niveau de la partie de face arrière du boîtier, un moyen d'émission de lumière (83) étant pourvu au niveau de la surface interne du couvercle de maintien de doigt (81), un moyen de réception de lumière (84), en tant que moyen de détection de pouls, étant pourvu au niveau de la face arrière du boîtier (11) à l'opposé du moyen d'émission de lumière (83).

24. Équipement électronique (1) selon la revendication 18, comprenant : un moyen d'affichage (13) servant à afficher un guide de commande ou d'information au niveau de la partie de face avant du boîtier externe (11), où un des moyens de détection de température est pourvu en tant que dit moyen de détection d'indicateur biométrique (101) au niveau de la partie de surface latérale par rapport à la partie de face avant du boîtier externe (11).

25. Équipement électronique (1) selon la revendication 18, comprenant : un moyen de commande de fonctionnement (12), où le moyen de détection de température d'extrémité de doigt en tant que dit moyen de détection d'indicateur biométrique (101) est pourvu à un emplacement avec lequel le doigt d'un utilisateur vient au contact de la surface du moyen de commande de fonctionnement (12).

26. Équipement électronique (1) selon la revendication 18, dans lequel le moyen de détection de température de la paume est pourvu au niveau de la partie du coin de la surface périphérique externe du boîtier (11).

27. Équipement électronique (1) selon la revendication 18, dans lequel une partie de détection (80) comprenant un couvercle de maintien de doigt (81) ayant une forme de surface interne incurvée de manière à prendre sensiblement la même forme que la forme de l'extrémité du doigt de l'utilisateur, et une partie d'insertion d'extrémité de doigt (82) formée entre le couvercle de maintien de doigt (81) et la face arrière du boîtier (11) sont pourvues au niveau de la partie de face arrière du boîtier, un moyen de détection de température d'extrémité de doigt, en tant que dit moyen de détection d'indicateur biométrique (101), étant pourvu au niveau de la partie de face arrière du boîtier (11).
